# EUROPEAN PATENT APPLICATION

(11) **EP 4 434 444 A1**
(43) Date of publication of application: **25.09.2024**
(21) Application number: 23164023.6
(22) Date of filing: 24.03.2023
(51) Int. Cl.: A61B 5/00, A61B 5/024, A61B 5/08, A61B 5/1455

(54) **DEVICE, SYSTEM AND METHOD FOR ASSESSING QUALITY OF A VITAL SIGN SIGNAL**

(71) Applicant: Koninklijke Philips N.V., 5656 AG Eindhoven (NL)
(72) Inventor: DEN BRINKER, Albertus Cornelis, Eindhoven (NL); KRISHNAN SANTHANA NAIDU, Navaneetha, Eindhoven (NL); BALMAEKERS, Benoît Marie, 5656AG Eindhoven (NL); ROCQUE, Mukul Julius, Eindhoven (NL)
(74) Representative: Philips Intellectual Property & Standards

(57) **Abstract**

The present invention relates to a device for assessing quality of a vital sign signal, the device comprising a signal input configured to obtain a vital sign signal indicating a non-invasively detected vital sign of a patient; a processing unit configured analyze the vital sign signal and to detect a disturbing signal of non-biological origin in the vital sign signal by identifying a deterministic signal contribution, wherein a variance of the deterministic signal contribution is less than a predetermined threshold variance; and an evaluation unit configured to determine a first quality metric for the vital sign signal based on the identification of the deterministic signal contribution. The present invention further relates to a corresponding method, system and computer program.

## Description

### FIELD OF THE INVENTION

The present invention relates to a device for assessing quality of a vital sign signal. The present invention further relates to a corresponding system and method.

### BACKGROUND OF THE INVENTION

Vital signs such as heart rate (HR), respiratory rate (RR), or blood oxygen saturation are important indicators of a person's health and well-being as well as predictors of acute medical conditions and chronic disease states. For this reason, vital signs are widely monitored in hospitalized and outpatient care settings, at home, or in other medical, leisure and fitness settings.

The Vital Signs Camera (VSC), for example, is a well-known concept and enables monitoring of one or more vital signs in unobtrusive ways by using a (remote) camera as prime sensor. As with any other monitoring technique also vital sign signals obtained by VSC may be distorted by noise. Noise comprises measurement noise, such as signal-to-ratio noise or noise caused by motion of a patient, or quantization noise, for example. In general, noise in a signal is characterized by its high unpredictability. Accordingly, it can only be described in stochastic terms.

Noise is a standard component in a quality metric of vital sign signals and the variation of a measurement caused by noise usually is kept within strong limits. In order to reduce noise in signals, there exists a wide variety of well-known reduction techniques.

However, vital sign signals obtained by VSC (or other monitoring equipment) may not only be distorted by noise, but also by disturbing signals originating from all kinds of environmental factors. For instance, the global illumination in a room may vary due to a cloud shifting in front of the sun, there may be (small) involuntary movements of the patient or the VSC may be subject to vibration. Special measures for mitigating these disturbances have been addressed as well, for example by digital image stabilization (*DIS) algorithms.*

Applications of the VSC and other patient monitors include their use in clinical environments. Clinical environments, being man-made and highly controlled, have specific conditions, creating certain peculiarities in lighting conditions. In particular, status and alarm indicators are ubiquitous stemming from the abundance of clinical devices in these environments. Accordingly, many disturbing signals appear here as a consequence of the presence of the abundance of medical (control) equipment and therefore the common characteristic of said disturbing signals is their "synthetic" nature.

In order to address these problems regarding disturbing signals typical for clinical environments patient imaging processing, particularly VSC processing, has to be improved.

In general, VSC processing and other image processing techniques are developed either model-based or data-driven. In case of data-driven processing, the clinical environments can best be a separate development case where on-the-spot training with intended clinical devices producing alarms or status updates must be taken along in the training, validation and testing processes. This can be different for each part of the clinical environment, e.g., ward specific. In model-based processing, the consequences of specific clinical conditions can be captured (partly) in the mechanisms, for example, by using signal-compensation techniques (as pre- or post-processing), or for DIS an inclusion into the core algorithm.

However, both model-based and data-driven approaches imply potential issues when the system is introduced into a new environment. It is in general cumbersome to retrain a data-driven model for each new environment as it typically requires new annotation. Similarly, it is undoable to foresee all possible disturbances in sufficient qualitative and quantitative detail to incorporate general measures in a model-based approach.

Accordingly, the multitude of status indicators and alarms within clinical scenarios poses a risk when introducing VSC devices into these environments. If exact knowledge of alarm/alert/status indicators was available, this potentially would open ways for compensation techniques. However, in the current clinical practice it is not expected that the signaling of all devices can be tapped into by access to, e.g., the general electronic hospital system. It is also questionable if this will come about in the foreseeable future as it would not only give rise to an extremely complicated infrastructure, it would also present a digital hazard in many ways.

### SUMMARY OF THE INVENTION

It is an object of the present invention to provide a device for vital signs (VS) monitoring that is more robust than existing systems to adverse environmental conditions when operated in a clinical environment. Accordingly, it is an object of the present invention to provide a device which allows for a more reliable vital sign monitoring, particularly in clinical environments.

In a first aspect of the present invention a device for assessing quality of a vital sign signal is presented. The device comprises:
a signal input configured to obtain a vital sign signal indicating a visually, non-invasively detected vital sign of a patient;
a processing unit configured analyze the vital sign signal and to detect a disturbing signal of non-biological origin in the vital sign signal by identifying a deterministic signal contribution, wherein a variance of the deterministic signal contribution is less than a predetermined threshold variance; and
an evaluation unit configured to determine a first quality metric for the vital sign signal based on the identification of the deterministic signal contribution.

In a second aspect of the present invention a system for assessing quality of a vital sign signal is presented, the system comprising:
a vital sign sensor configured to sense a vital sign of a patient in a visual, non-invasive manner and to generate a vital sign signal based on the sensed vital sign;
a device for assessing quality of a vital sign signal; and
an alarm unit configured to obtain an alarm signal from said device and to trigger an alarm if the alarm signal is obtained.

In yet further aspects of the present invention, there are provided corresponding methods, a computer program which comprises program code means for causing a computer to perform the steps of the methods disclosed herein when said computer program is carried out on a computer as well as a non-transitory computer-readable recording medium that stores therein a computer program product, which, when executed by a processor, causes the methods disclosed herein to be performed.

Preferred embodiments of the invention are defined in the dependent claims. It shall be understood that the claimed system, method and computer program have similar and/or identical preferred embodiments as the claimed device and as defined in the dependent claims and as disclosed herein.

As described above, current VSC processing, both data-driven and model-based, is not as reliable as desired. There is a lack of possibilities of adequate upfront arrangements and tuning of VSC systems for new environments and retraining or model development for new environments is cumbersome. However, clinical environments have specific conditions allowing to counter said problems. In fact, clinical environments have certain peculiarities in lighting conditions. Status and alarm indicators, for example, are ubiquitous stemming from the abundance of clinical devices. Furthermore, said indicators by visual cues have certain specific qualities, particularly stemming from their specific function to attract attention of medical staff. More specifically, the induced effects of said clinical indicators separate them from "naturally" occurring phenomena, i.e. light emitted and/or reflected by biological sources such as the sun or patients. Accordingly, said indicators and their effects have a so-called synthetic character. In other words, light emitted from clinical devices may generally be represented by highly deterministic signals, whereas light of biological origin or manipulated by a biological subject is represented by less deterministic or even stochastic signals.

In fact, in most cases the disturbing signals reveal machine-produced patterns, i.e. patterns of little variation. Therefore, disturbing contributions in a vital signs signal obtained in a clinical environment manifest themselves in well-defined (synthetic) oscillation or repetition, a hard on/off switch, a very well-defined color change (i.e. a very well-defined color direction in color space) or the like.

These qualitative differences between natural occurring signals and the peculiar disturbances encountered in clinical environments are sufficient to enable a new type of screening, i.e. quality check, of the VSC/imaging processing output. The existing screening is typically a form of quality metric where it is checked if the output signal (or signals in intermediate processing steps) adhere to certain desired properties.

Contrary to existing quality screenings the approach proposed herein consists of screening for certain undesired properties as associated with dominant disturbing signals known to be present in clinical environments.

The newly introduced (first) quality metric allows to introduce existing VSC or other imaging technology in such environments, with i) limited risk of producing erroneous output and ii) possibilities to check the need for retraining/redesigning the system. The latter would arise only if the screening would indicate that the occurrence of low quality due to disturbances is that frequent that meaningful use in clinical practice is jeopardized.

According to the present invention disturbing signals originating from clinical equipment or associated with said specific clinical environment reveal specific features which allow for further improvement of imaging processing in these environments.

According to the first aspect of the present invention said improvement is achieved by analyzing the vital sign signal corresponding to a patient for signal parts of synthetic origin, i.e. for parts of the signal which are more regular than expected. In other words, there is proposed a device configured to search for deterministic signal contributions in the vital sign signal (particularly intervals in a signal in the time domain) since such contributions indicate a non-biological origin and may stem from lights of surrounding medical devices. Although even signals stemming from such devices may not be perfectly deterministic, the variance of these signals is extremely little qualifying them as deterministic signals. In this context, the variance is a metric describing the (strength and/or frequency of) deviations of a signal from a (expected, perfectly) deterministic signal. For example, the variance describes the (strength and frequency of) deviations of an almost periodic signal with respect to a perfectly periodic signal. More specifically, the variance of a signal may be defined as the square of the standard deviation of the signal. Particularly, the variance could relate to the variance of a determined rate (i.e. dominant frequency) of a (time-series) signal or a determined amplitude (i.e. dominant amplitude) of a (time-series) signal. The variance of a signal may likewise be a deviation of a fit of a measured signal from the expected disturbance signal, such as a sinusoidal or block wave.

In general, the term "variance" may be understood as an error measure of the deterministic signal contribution from a truly deterministic signal. Hence, the term variance may likewise be a mean squared error, a mean absolute error, a maximum deviation, a mean absolute percentage error or a root mean square error of the detected deterministic signal contribution from a truly deterministic signal. The truly deterministic signal may be predetermined.

It is proposed to inspect the obtained vital sign signal (e.g., the pulse rate trace) or an intermediate signal from the VSC processing with respect to its variance, particularly with respect to the variance of contributing signal parts. In general, it is expected that the variance of a "natural" signal is higher than the variance of almost perfectly deterministic signals originating from machines. Hence, if the variance of a signal contribution (particularly an interval of a time-series signal) is lower than an expected value (predetermined threshold variance), said signal contribution can be associated to a non-biological origin and the first quality metric of the corresponding vital sign signal can be set to a low value. On the other hand, if the variance of the obtained signal is as expected and, in particular, the same or higher as the predetermined threshold variance, the first quality metric can be set to a high value.

In particular, the evaluation unit may be configured to determine the first quality metric based on the difference between the variance of the deterministic signal contribution and the predetermined threshold variance. In fact, the higher the difference, the higher would be the likelihood for the deterministic signal contribution to indeed be of non-physiological origin. Accordingly, the higher the difference, the lower the first quality metric may be set.

Therefore, the device has means for detecting synthetic signals which are an intrinsic part of clinical environments like hospitals wards and means for incorporating this in the quality metric.

As to the units of the device, the signal input may be directly or indirectly (e.g. via a network or bus) coupled or connected to a vital sign sensor or a storage providing a vital sign signal. The input unit may thus e.g. be a (wired or wireless) communication interface or data interface, such as a Bluetooth interface, Wi-Fi interface, LAN interface, HDMI interface, direct cable connection, or any other suitable interface allowing data transfer to the device for assessing quality of a vital sign signal.

The processing unit may be any kind of means configured to process the obtained vital sign signal and to analyze said signal. It may be implemented in software and/or hardware, e.g. as a programmed processor, computer, laptop, PC, workstation, etc. The processing unit may be configured to detect/identify one or more deterministic signal contributions. Furthermore, the processing unit may be configured to filter the vital sign signal (prior to the detection of a deterministic signal contribution). In particular, the processing unit may be configured to filter noise from the vital sign signal.

The evaluation unit may be any kind of means configured to process information regarding existence (and strength) of a deterministic signal contribution in the vital sign signal. Accordingly, the evaluation unit may be implemented in software and/or hardware, e.g. as a programmed processor, computer, laptop, PC, workstation, etc.

The evaluation unit may be configured to determine the first quality metric, more precisely to set a value for the first quality metric, based on the (strength and/or number) of identified deterministic signal contribution(s). The (values of the) quality metric can either be represented by a discrete or a continuous spectrum. Accordingly, the value of the first quality metric may be set (i.e. determined to be) the lower the higher the number of deterministic signal contributions and/or the higher the strength of the one or more deterministic signal contributions is. The quality metric is thus descriptive of the quality of the vital sign signal.

The evaluation unit may also be configured to output the (set value of) the first quality metric. Hence, the evaluation unit may further comprise an interface that provides the (value of the) first quality metric, e.g. transmits it to another device or provides it for retrieval by another device, e.g. transmits it to another computer or transfers it directly to as display for displaying it. The interface of the evaluation unit may generally be any (wired or wireless) communication or data interface. However, instead of the evaluation unit being configured to output the first quality metric itself the device may further comprise a separate output unit to output the first quality metric.

The deterministic signal contribution may comprise any of a deterministic frequency contribution, particularly a repetitive frequency pattern, and/or a deterministic wavelength contribution (i.e. color contribution), for example a very-well defined change of color. In fact, a deterministic frequency contribution may indicate a blinking light blinking at a constant rate, for example. For a photoplethysmographic signal (PPG signal) there exists an expected color direction in color space (i.e. an expected course of color change in the signal) relating to the varying hemoglobin spectrum of the patient. Hence, a change of light in the signal caused by a synthetic light source such as an LED in the patient's room, for example, may lead to unexpected, normalized amplitudes in R, G and B signal channels of an RGB signal. Particularly, disturbances from e.g. red or green indicator LEDs from surrounding equipment would show as dominant amplitudes in said signal channels.

The introduced quality metric, being associated with undesired properties of the signal, may be used as stand-alone quality check for vital sign monitoring or may be combined with existing screening methods, particularly with other quality metrics.

According to an embodiment, the vital sign signal obtained by the signal input comprises a superposition of the true vital sign signal and the deterministic signal.

For example, in case the heart rate of a patient is monitored via remote photoplethysmography using a camera the vital sign signal comprises a heart-rate signal which may be a superposition of a signal representing the true heart rate and a light signal originating from a blinking LED of a medical device near the patient. In fact, the light signal may dominate the resulting heart rate signal obtained by the input unit and hence lead to wrong interpretations with respect to the heart rate of the patient. Similarly, the light signal may appear as repetitive intermediate signal in the resulting heart-rate signal.

In another example, in the case of PPG monitoring via VSC a blinking flashed green light may overlap with a PPG color signature (dominant green) and may have a repetition rate within the heartbeat range (e.g., 1 Hz). Accordingly, when picked up by the VSC processing a signal comprising an unnaturally stable, repetitive time pattern will be found. The signal part originating from blinking flashed green light can be identified by the processing unit and the value of the first quality metric can be set to a low value.

According to another embodiment, the processing unit is configured to analyze the vital sign signal in the time domain and/or in the frequency domain. In particular, the processing unit may be configured to transform a vital sign signal in the time domain into a vital sign signal in the frequency domain and vice versa. In particular, in cases where heart rate, pulse rate or other repetitive vital signs are monitored an analysis of the vital sign signal in the frequency domain may offer advantages in terms of analyzing the frequency composition of the signal, particularly in terms of identifying dominating frequencies, for example.

According to another embodiment, the processing unit is configured to split the vital sign signal into constituent frequencies and/or wavelengths to identify the deterministic signal contribution. In particular, the processing unit may be configured to perform a harmonic analysis or a Fourier transformation of the vital sign signal. Knowledge of the constituent frequencies (and their strength) of the vital sign signal may help identify signals of non-biological origin. This is due to the fact that lights of clinical devices are often lights flashing at fixed frequencies. Furthermore, some lights, such as LEDs, are often pulse-width modulated. Information about the constituent wavelengths may be obtained by a spectral analysis. Similarly, in the case of an RGB signal the intensities of the different color contributions may be analyzed. In fact, lights of clinical devices are often color-coded to indicate emergency. Particularly, lights in red, orange or green are often used in clinical devices. Hence, a signal contribution corresponding to the wavelengths/frequencies of these lights may be identified as synthetical.

In another embodiment, the processing unit is configured to detect the disturbing signal by identifying an amplitude of a frequency component, particularly an amplitude of a harmonic component, to be above a predetermined threshold. For example, the normal resting heart rate of a human ranges from 60 to 100 beats per minute (bpm). Hence, in case of heart rate monitoring, if the processing unit detects that the amplitude of the frequency component corresponding to 50 beats per minute, for example, is higher than expected, i.e. higher than a predetermined threshold, said signal component can be associated with non-human origin (and in the case of a clinical environment with non-biological origin).

In yet another embodiment, the deterministic signal contribution comprises a periodic signal, particularly a constant signal, a sinusoidal signal, a block wave (i.e. square wave) signal, a triangle wave signals, a sawtooth wave signals or any combinations of said signals. However, the deterministic signal contribution may likewise comprise a pulse train signal.

A periodic signal repeats itself after a fixed time period. Therefore, constant signals are regarded as periodic signals here in this application. A sinusoidal signal is a periodic signal which is based on the sine or cosine function from trigonometry. Regularly blinking lights in medical devices may produce sinusoidal or block wave signals, for example. Furthermore, highly sinusoidal signal contributions in PPG signals may be caused by AC (alternating current) interference. One notable way for AC interference to affect PPG measurements is when illumination (of incandescent of fluorescent lamps) are modulated by AC of equipment used and in turn modulate the recorded PPG signal (i.e. light/image captured by a PPG sensor). Furthermore, while LED illumination is generally less affected by AC frequencies, often pulse width modulation (PWM) is used for dimming LEDs. Accordingly, PWM may have a similar effect on a measured PPG signal. In general, deterministic signal contributions caused by AC and PWM may be distinguished by their frequencies (generally, PWM frequencies are much higher than AC frequencies).

In fact, visuals of medical devices may mostly be characterized as very steady repeated patterns such as lights flashing at repeated intervals, i.e. with regular patterns.

In a further embodiment, the vital sign signal comprises any of a heart rate signal; a heart rate variability signal; a photoplethysmography, PPG, signal; a breathing rate signal; and a respiration rate signal.

In yet another embodiment, if the vital sign signal is the heart rate signal, the predetermined threshold variance is 10 (beats per minute)^2, preferably 4 (beats per minute)^2, more preferably 1 (beat per minute)^2, and/or if the vital sign signal is the heart rate variability signal, the predetermined threshold variance is 30 (milliseconds)^2, preferably 10 (milliseconds)^2, more preferably 1 (milliseconds)^2, and/or if the vital sign signal is the breathing rate signal, the predetermined threshold variance is 1 (breaths per minute)^2, preferably 0.1 (breaths per minute)^2, more preferably 0.01 (breaths per minute)^2.

In another embodiment, the evaluation unit is configured to provide a first alarm signal if the first quality metric does not comply with a predetermined first quality metric threshold. To be more precise, the evaluation unit is configured to provide a first alarm signal if the value set for the first quality metric does not comply with a predetermined first quality metric threshold.

For example, if the vital sign signal (or intermediate signals thereof from the VSC processing) comprises a deterministic signal contribution, i.e. a contribution comprising a variance which is less than a predetermined threshold variance, the (value of the) first quality metric is compared to a first quality metric threshold. If the (value of the) first quality metric does not comply with said threshold, the evaluation unit is configured to trigger an alarm signal. This way, a warning or a low reliability indication can be generated in case the outcome of the quality analysis tends to the character of a synthetic signal so that medical staff can be warned that the vital sign signal is most likely not reliable.

In yet another embodiment, the processing unit is configured to correct the vital sign signal based on the first quality metric. In particular, the identified deterministic signal contributions may be deleted in the vital sign signal, particularly if the (value of the) first quality metric does not comply with the predetermined first quality metric threshold. The processing unit may likewise be configured to suppress an output of the vital sign signal if the (value of the) first quality metric does not comply with the predetermined first quality metric threshold. Similarly, if the (value of the) first quality metric does not comply with the predetermined first quality metric threshold the vital sign signal may be labelled as unreliable.

In a further embodiment, the evaluation unit is configured to combine the first quality metric with a second quality metric; wherein the processing unit is configured to correct the vital sign signal based on the combination of the first quality metric and the second quality metric and/or wherein the evaluation unit is configured to provide a second alarm signal if the combination of the first quality metric and the second quality metric does not comply with a predetermined combined quality metric threshold.

In other words, the evaluation unit may be configured to combine the value of the first quality metric with a value of a second quality metric; wherein the processing unit is configured to correct the vital sign signal based on the combination of said values and/or wherein the evaluation unit is configured to provide a second alarm signal if the combination of said values does not comply with a predetermined combined quality metric threshold. The (value of the) second quality metric may be determined/set by the evaluation unit itself prior to or before the determination of (the value of) the first quality metric. However, the evaluation unit may alternatively obtain the (value of the) second quality metric from another entity.

The second quality metric may indicate, for example, the signal-to-noise ratio of the vital sign signal, i.e. the second quality metric may indicate to what extent the vital signal is affected by noise, i.e. stochastic disturbances. Similarly, the second quality metric may indicate whether (or to what extend) the vital sign signal adheres to a desired property, such as a desired frequency range, for example. In fact, vital sign signals are usually analyzed with respect to desired properties, wherein a quality metric of the signal represents if (and to what extend) the signal complies with the expected properties. The second quality metric may be a number or a category and may be further used to label the vital sign signal, particularly in terms of reliability. For example, (when displayed) the vital sign signal could be color coded with red signal indicating a non-trustworthy signal. Apart from that the (value of the) second quality metric may be used by the processing unit to adapt the vital sign signal. For example, if the second quality metric is below a predetermined threshold, the processing unit may be configured to suppress an output of the vital sign signal. In other words, the units of the device may handle the second quality metric (or a combination of the first and the second quality metric) in the same way as the first quality metric.

In general, the signal input of the device disclosed may be configured to obtain a plurality of vital sign signals indicating vital signs of a patient as recorded from different spatial regions of a patient's body. For example, the device may obtain a plurality of vital sign signals corresponding to a plurality of body regions, the signals generated by using a plurality of images showing different body parts of the patient. In that case, the invention proposed may be applied in parallel to said different signals and a signal from regions dominantly containing non-physiological disturbances may not be included in an aggregated signal. However, signals from such regions with dominant disturbances may be used as input to filtering steps in signals from other regions, where the same disturbances may not be dominant, but still present and affecting the signal quality. On the other hand, for the input to filtering in the processing unit, also signals from regions not covering the patient may be used (e.g. to obtain the most accurate estimation of the non-physiological disturbance (i.e. deterministic signal contribution), and to prevent the risk of vital signs leaking into the reference signal which may lead to the vital signs signal(s) being damped as well by the filtering).

In an embodiment of the system for assessing quality of a vital sign signal the vital sign sensor comprises a camera configured to acquire images of the patient in a visible spectrum portion and/or in an infrared spectrum portion. The images may particularly comprise video images. The camera configured to acquire images of the patient in the visible spectrum may be an RGB camera configured to capture images by capturing light in red, green, and blue wavelengths (RGB). The camera configured to acquire images of the patient in the infrared spectrum may particularly acquire images in the mid- and/or long wavelength infrared spectrum (i.e. may be a thermal camera).

A classical embodiment of a vital sign sensor is a vital signs camera (VSC; i.e. a device for determining vital signs of a subject), particularly a VSC for remote PPG. The VSC for remote PPG uses a reflective method of measurement of a photoplethysmography (PPG) signal, where an ambient or dedicated illumination is used. PPG generally refers to the measurement of volume changes of an organ or a body part and in particular to the detection of volume changes due to a cardio-vascular pulse wave traveling through the body of a subject with every heartbeat.

PPG is an optical measurement technique that evaluates a time-variant change of light reflectance or transmission of an area or volume of interest. It is based on the principle that blood absorbs light more than surrounding tissue, so variations in blood volume with every heartbeat affect transmission or reflectance correspondingly. Besides information about the heart rate, a PPG waveform can comprise information attributable to further physiological phenomena such as respiration. By evaluating the transmissivity and/or reflectivity at different wavelengths (typically red and infrared), the blood oxygen saturation can be determined.

Remote PPG utilizes light sources or, in general radiation sources, disposed remotely from the subject of interest (patient). Similarly, also a detector/sensor, e.g., a camera, such as the VSC, can be disposed remotely from the patient. The remote PPG sensor is essentially a camera with signal processing logic which estimates a PPG signal from the images (video sequence).

Irrespective of the type of vital sign sensor, said sensor is configured to transmit the vital sign signal to another device or to provide it for retrieval by another device. It may thus comprise any (wired or wireless) communication or data interface.

Concerning the alarm unit of the system, said unit may be directly or indirectly (e.g. via a network or bus) coupled or connected to the device for assessing the quality of a vital sign signal. The alarm unit may thus comprise e.g. a (wired or wireless) communication interface or data interface, such as a Bluetooth interface, Wi-Fi interface, LAN interface, HDMI interface, direct cable connection, or any other suitable interface allowing data transfer, particularly transfer of the alarm signal, from the device for assessing the quality of the vital sign signal.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a device according to the present invention,
Fig. 2 shows an illustration of pulse rate signal as obtained from a vital signs camera according to the present invention and as obtained from a finger probe,
Fig. 3 shows a schematic diagram of a first embodiment of a system according to the present invention,
Fig. 4 shows an illustration of a second embodiment of a system according to the present invention,
Fig. 5 shows a flow chart of a first embodiment of a method according to the present invention, and
Fig. 6 shows a flow chart of a second embodiment of a method according to the present invention.

### DETAILED DESCRIPTION OF THE INVENTION

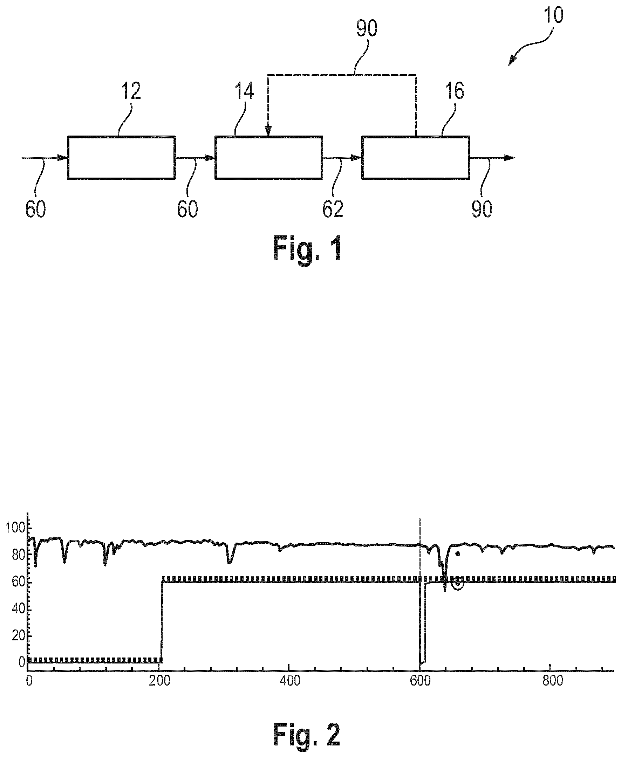

Fig. 1 shows a schematic diagram of an embodiment of a device 10 according to the present invention. The device comprises a signal input 12, a processing unit 14 and an evaluation unit 16.

The signal input 12 is configured to obtain a vital sign signal 60 such as a PPG signal indicating the heart rate of a patient. In the processing unit 14 the vital sign signal 60 may be filtered to reduce noise in the signal. The filtered vital sign signal 60 may then be analyzed for contribution of a disturbing signal 70 of non-biological origin. To this end, the processing unit 14 may be configured to detect a deterministic signal contribution, i.e. a signal contribution comprising less variance than an expected lowest variance (i.e. a predetermined threshold variance). Information about the identification of a deterministic signal contribution 62 may then be provided to the evaluation unit 16 which is configured to determine a first quality metric 90 for the vital sign signal 60 based on said information. In particular, the evaluation unit 16 may set the (value of the) first quality metric 90 to a low value if a deterministic signal contribution and hence a disturbing signal of non-biological origin is detected and to a high value if no deterministic signal contribution is detected.

The quality metric 90 may then be output by the evaluation unit 16 and/or may be provided to the processing unit 14 to adapt the vital sign signal 60. In particular, the processing unit 16 may be configured to delete the identified deterministic signal contribution(s) from the vital sign signal 60.

Fig. 2 shows an illustration of pulse rate signal as obtained from a vital signs camera according to the present invention and as obtained from a finger probe. The pulse rate as obtained from the vital signs camera is illustrated by a dotted line whereas the pulse rate as obtained from finger probe is represented by a solid line. As can be seen from the graph both signals differ significantly. The pulse rate obtained from the finger probe is illustrated as reference pulse rate and represents the true pulse rate of a patient. From this reference signal it can be derived that the pulse rate of the patient more or less amounts to 90 bpm. There can also be seen a significant variance of the signal, which is typical for pulse rate measurements. The pulse rate according to the pulse rate signal as obtained from the vital signs camera is either zero or 60 and the variance of both contributions is almost zero and particularly less than typically expected for a pulse rate signal. Therefore, said signal can be associated with non-biological origin, particularly with a LED of a clinical device blinking at 1 Hz, for example, in the environment of the patient. In other words, the dominating frequency of changing illumination of said LED causes the pulse rate as obtained by the camera to be stuck at 60 bpm.

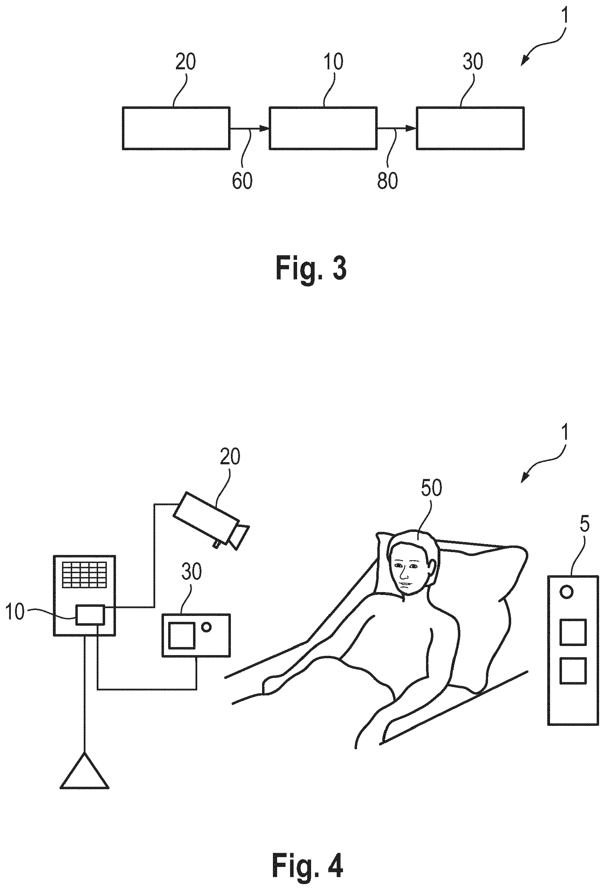

Fig. 3 shows a schematic diagram of a first embodiment of a system 1 according to the present invention. The system 1 comprises a vital sign sensor 20, a device 10 for assessing the quality of a vital sign signal and an alarm unit 30.

The vital sign sensor 20 may be a camera, such as the VSC, which is disposed remotely from the patient to sense a vital sign of the patient in a non-invasive manner and to generate a vital sign signal 60 based on the sensed vital sign. The camera then provides the vital sign signal 60 to the device 10. The device 10 is configured to determine whether the vital sign signal comprises a disturbing signal of non-biological origin. If the device 10 determines such a signal, particularly a deterministic signal contribution, it determines a first quality metric for the vital sign signal 60. Based on said quality metric an alarm signal 80 may be generated. In particular, if it is determined by the device 10 that the vital sign signal 60 comprises a deterministic signal contribution the first quality metric may be set to a value below a (predetermined) first quality metric threshold. However, if the quality metric exhibits a value below the first quality metric threshold the evaluation unit 16 of the device 10 may be configured to generate a first alarm signal 80 and to provide said first alarm signal 80 to the alarm unit 30 of the system 1. If the alarm unit 30 obtains the first alarm signal 80, the alarm unit is configured to trigger an alarm. Said alarm may be a sound alarm or a visual alarm. For example, the alarm may be provided on a display.

Fig. 4 shows an illustration of a second embodiment of a system 1 according to the present invention. The illustration shows a clinical environment with a patient 50 being observed by a vital sign sensor 20, which in this case is a vital signs camera. The vital signs camera is configured to detect a vital sign of the patient 50 in a non-invasive, remote manner and to generate a vital sign signal based on the sensed vital sign. However, the vital signs camera may not only sense light reflected by the patient as desired but may also catch light from a light source of the medical device 5. The light emitted by the medical device may shine in a continuous manner or may blink at regular intervals. The vital sign signal (comprising the light signal from the medical device 5) may then be provided to the device 10 for assessing the quality of the vital sign signal. In this embodiment, the device 10 is connected to the vital signs camera in a wired manner. However, the vital sign sensor 20 and the device 10 may likewise be connected wirelessly. The system 1 illustrated further comprises an alarm unit 30 which is part of a medical device and connected to the device 10 in a wired manner. Similarly, the device 10 and the alarm unit 30 may be connected wirelessly.

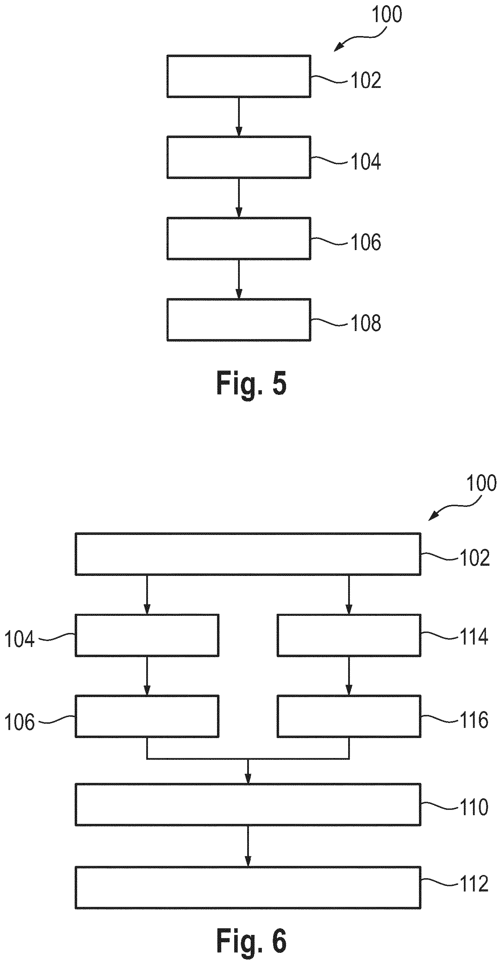

Fig. 5 shows a flow chart of a first embodiment of a method 100 according to the present invention. In a first step 102 of the method 100 a vital sign signal indicating a non-invasively detected vital sign of a patient is obtained. In a second step 104 the vital sign signal may be analyzed with respect to a disturbing signal of non-biological origin by determining whether the vital sign signal comprises a deterministic signal part/contribution, the deterministic signal part being characterized by the variance of the deterministic signal contribution being less than a predetermined threshold variance, i.e. a predetermined lowest expected variance. Subsequently, in step 106 a first quality metric for the vital sign signal is determined based on the identification of the deterministic signal contribution. In particular, if there is found a deterministic signal contribution in step 104 the first quality metric may be assigned to a value lower than a first quality metric threshold, the specific value of the first quality metric depending on the strength of the deterministic signal contribution. On the other hand, if no such signal contribution is found in step 104 the first quality metric may be assigned to a value higher than the first quality metric threshold. In a last step 108 the determined quality metric may be output.

Fig. 6 shows a flow chart of a second embodiment of a method according to the present invention. In a first step 102 a vital sign signal is obtained. The vital sign signal may then be analyzed in two different ways. In step 104 the vital sign signal may be analyzed with respect to a deterministic signal contribution indicating a disturbing signal originating from non-biological sources, such as medical machines. Hence, in step 104 the vital sign signal is analyzed with respect to an undesired property. In parallel, in step 114 the vital sign signal may be analyzed with respect to a desired property. For example, in case of a heart rate signal it is determined whether the heart rate signal is within predetermined limits of the heart rate. For both analyses in steps 104 and 114 a quality metric is determined in steps 106 and 116. In other words, in step 104 a first quality metric based on the identification of the deterministic signal contribution is determined whereas in step 106 a second quality metric based on the determination, whether the heart rate signal is within said predetermined limits, is determined. The first quality metric and the second quality metric are then combined to a combined quality metric in step 110. In step 112 the combined quality metric is used to correct the vital sign signal and/or to generate an alarm signal.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Device (10) for assessing quality of a vital sign signal (60), the device (10) comprising:
a signal input (12) configured to obtain a vital sign signal (60) indicating a visually, non-invasively detected vital sign of a patient (50);
a processing unit (14) configured analyze the vital sign signal (60) and to detect a disturbing signal (70) of non-biological origin in the vital sign signal by identifying a deterministic signal contribution, wherein a variance of the deterministic signal contribution is less than a predetermined threshold variance; and
an evaluation unit (16) configured to determine a first quality metric for the vital sign signal (60) based on the identification of the deterministic signal contribution.

2. Device (10) as claimed in claim 1,
wherein the vital sign signal (60) obtained by the signal input (12) comprises a superposition of the true vital sign signal (62) and the deterministic signal contribution.

3. Device (10) as claimed in claim 1 or 2,
wherein the processing unit (14) is configured to analyze the vital sign signal (60) in the time domain and/or in the frequency domain.

4. Device (10) as claimed in any of the preceding claims,
wherein the processing unit (14) is configured to split the vital sign signal (60) into constituent frequencies and/or wavelengths to identify the deterministic signal contribution.

5. Devices (10) as claimed in claim 4,
wherein the processing unit (14) is configured to detect the disturbing signal (70) by identifying an amplitude of a frequency component to be above a predetermined threshold.

6. Device (10) as claimed in any of the preceding claims,
wherein the deterministic signal contribution comprises a periodic signal, particularly a constant signal and/or a sinusoidal signal; a pulse train signal and/or a block wave signal.

7. Device (10) as claimed in any of the preceding claims,
wherein the vital sign signal (60) comprises any of a heart rate signal; a heart rate variability signal; a photoplethysmography, PPG, signal; a breathing rate signal; and a respiration rate signal.

8. Device (10) as claimed in claim 7,
wherein, if the vital sign signal (60) is the heart rate signal, the predetermined threshold variance is 10 (beats per minute)^2, preferably 4 (beats per minute)^2, more preferably 1 (beat per minute)^2, and/or
if the vital sign signal (60) is the heart rate variability signal, the predetermined threshold variance is 30 (milliseconds)^2, preferably 10 (milliseconds)^2, more preferably 1 (milliseconds)^2, and/or
if the vital sign signal (60) is the breathing rate signal, the predetermined threshold variance is 1 (breaths per minute)^2, preferably 0.1 (breaths per minute)^2, more preferably 0.01 (breaths per minute)^2.

9. Device (10) as claimed in any of the preceding claims,
wherein the evaluation unit (14) is configured to provide a first alarm signal (80) if the first quality metric does not comply with a predetermined first quality metric threshold.

10. Device (10) as claimed in any of the preceding claims,
wherein the processing unit (14) is configured to correct the vital sign signal (60) based on the first quality metric.

11. Device (10) as claimed in any of the preceding claims,
wherein the evaluation unit (16) is configured to combine the first quality metric with a second quality metric;
wherein the processing unit (14) is configured to correct the vital sign signal (60) based on the combination of the first quality metric and the second quality metric, and/or wherein the evaluation unit (16) is configured to provide a second alarm signal (82) if the combination of the first quality metric and the second quality metric does not comply with a predetermined combined quality metric threshold.

12. System (1) for assessing quality of a vital sign signal, the system comprising:
a vital sign sensor (20) configured to sense a vital sign of a patient in a visual, non-invasive manner and to generate a vital sign signal (60) based on the sensed vital sign;
a device (10) as claimed in any of claims 1 to 11; and
an alarm unit (30) configured to obtain an alarm signal (80; 82) from said device (10) and to trigger an alarm if the alarm signal is obtained.

13. System (1) as claimed in claim 12,
wherein the vital sign sensor (20) comprises a camera configured to acquire images of the patient in a visible spectrum portion and/or in an infrared spectrum portion.

14. Method for assessing quality of a vital sign signal, the method comprising:
obtaining a vital sign signal indicating a visually, non-invasively detected vital sign of a patient;
analyzing the vital sign signal and detecting a disturbing signal of non-biological origin in the vital sign signal by identifying a deterministic signal contribution, wherein a variance of the deterministic signal contribution is less than a predetermined threshold variance; and
determining a first quality metric for the vital sign signal based on the identification of the deterministic signal contribution.

15. Computer program comprising program code means for causing a computer to carry out the steps of the method as claimed in claim 14 when said computer program is carried out on the computer.
